# EUROPÄISCHE PATENTSCHRIFT

(11) **EP 0 393 533 B1**
(45) Veröffentlichungstag und Bekanntmachung des Hinweises auf die Patenterteilung: **25.08.1993**
(21) Anmeldenummer: 90107107.6
(22) Anmeldetag: 12.04.1990
(51) Int. Cl.: A61M 16/18

(54) **Narkosemittelverdunster mit temperaturkompensierender Drosselvorrichtung**
Vaporizer for anaesthetic solutions with a temperature compensating throttle device
Vaporiseur pour solutions anesthésiques muni d'un dispositif d'étranglement compensateur de température

(30) Priorität: 19.04.1989 DE 3912818
(43) Veröffentlichungstag der Anmeldung: 24.10.1990
(73) Patentinhaber: Drägerwerk Aktiengesellschaft, 23542 Lübeck (DE)
(72) Erfinder: Wallroth, Carl Friedrich, Dr., D-2400 Lübeck (DE); Falb, Wolfgang, D-2401 Krummesse (DE); Mohr, Helmut, D-2406 Stockelsdorf (DE)

(56) Entgegenhaltungen:
- FR-A- 2 301 270
- GB-A- 1 224 478
- US-A- 4 879 997

## Beschreibung

Die Erfindung betrifft einen Narkosemittelverdunster mit einer Drosselung des ihn durchströmenden Gases, bei welcher ein Drosselspalt in Abhängigkeit von der Umgebungstemperatur durch eine Kompensationseinrichtung verstellbar ist, welche einen Stangenkörper aus Material mit geringem Wärmeausdehnungskoeffizienten besitzt, der in einer Hülse aus Material mit großem Wäremausdehnungskoeffizienten aufgenommen ist, wobei der Stangenkörper an seinem einen Ende mit der Hülse formschlüssig verbunden und an seinem anderen Ende mit einem Formkörper, der zusammen mit einem Gegenformkörper den Drosselspalt bildet, versehen ist.

Ein derartiger Narkosemittelverdunster ist aus der DE-PS 25 07 261 bekannt. Es handelt sich hierbei um einen Verdunster mit einem der Verdunsterkammer parallel geschalteteten Bypass. Um die Menge des verdunsteten Narkosemittels möglichst unabhängig von der Temperatur des Gerätes zu halten, wird die durch den Bypass strömende Gasmenge durch Verändern eines Drosselspaltes in Abhängigkeit von der Temperatur des Gerätes geregelt. Dabei wird zur Regelung der Drosselspaltbreite die hohe thermische Ausdehnung von miteinander verbundenen Gehäuseteilen sowohl der Verdunsterkammer als auch der an die Verdunsterkammer angeschlossenen Einstellvorrichtung ausgenutzt. Durch Verbindung mit Bauteilen mit geringem thermischen Ausdehnungskoeffizienten ergibt sich eine temperaturabhängige Drosselspaltbreite. Die Gehäuseteile sind aus Messing gefertigt und haben eine sehr komplizierte Formgebung mit engen Innenkonturen.

Da Narkosemittel sehr aggressiv sind, muß das Gehäuse gegen Korrosion durch z. B. Vernickeln geschützt werden. Aufgrund der komplizierten Geometrie der Narkosegas führenden Bauteile gelingt dies aber nur mangelhaft. Die den Drosselspalt bildenden Bauteile erfahren darüberhinaus durch ein Vernickeln unterschiedliche, in ihrer Dicke, gemessen an den geforderten engen Toleranzen, nicht hinreichend gleichmäßige Auftragungen, so daß eine Dosierung nur schwer mit der notwendigen Genauigkeit gewährleistet werden kann.

Aufgabe der Erfindung ist es, eine thermische Verstelleinrichtung eines Drosselspaltes anzugeben, bei der einfach gestaltete Teile eingesetzt werden können, deren Material hinsichtlich günstiger thermischer Ausdehnungskoeffizienten ausgewählt werden kann, ohne daß Rücksicht auf Korrosionsbeständigkeit genommen werden muß.

Die Aufgabe wird dadurch gelöst, daß die Hülse von einer Umhüllung aus korrosionsbeständigem Material umgeben ist, welche gegenüber den das Narkosegas führenden Durchströmungskanälen gasdicht abgeschlossen ist, und daß die Hülse an ihrem dem Formkörper zugewandten Ende auf einer Hülsenabstützung der Umhüllung ruht.

Der Vorteil der Erfindung liegt im wesentlichen darin, daß lediglich die einfach geformte Umhüllung mit dem korrosiven zu dosierenden Gas, wie z.B. Narkosemittel, in Berührung kommt, die leicht korrosionsbeständig, z.B. durch Vernickeln, gemacht werden kann, oder auch selbst aus korrosionsfestem Material besteht. Die den Drosselspalt bildenden Formkörper haben im Hinblick auf eine Temperaturkompensation mit der Bildung eines der veränderten Temperatur angepaßten Drosselspaltes nichts mehr zu tun, so daß deren Material ebenfalls lediglich in bezug auf gute Korrosionsbeständigkeit und auf paßgenaue Fertigbarkeit ausgesucht zu werden brauchen. Durch die "schwimmende" Lagerung der Stange in der Hülsenabstützung, miteinander verbunden nur an dem dem Formkörper abgewandten Ende, erzielt man eine gute Ausnutzung der Längenausdehnung in bezug auf genau veränderliche Spaltweiten.

Als Material mit geringem Wärmeausdehnungskoeffizienten wird vorzugsweise eine Nickel-Eisen-Legierung (Invar) verwandt. Als Material mit hohem Wärmeausdehnungskoeffizienten wird vorzugsweise Messing verwandt.

Die Empfindlichkeit der thermischen Regelung des Drosselspaltes kann durch Verlängerung der Stangen und Hülsen erfolgen. Andererseits ist es auch möglich dieses Ziel zu erreichen, indem auch der obere Ring mit einer äquivalenten thermischen Verstellung, gebildet aus miteinander verbundenen Stangen und Hülsen, versehen wird.

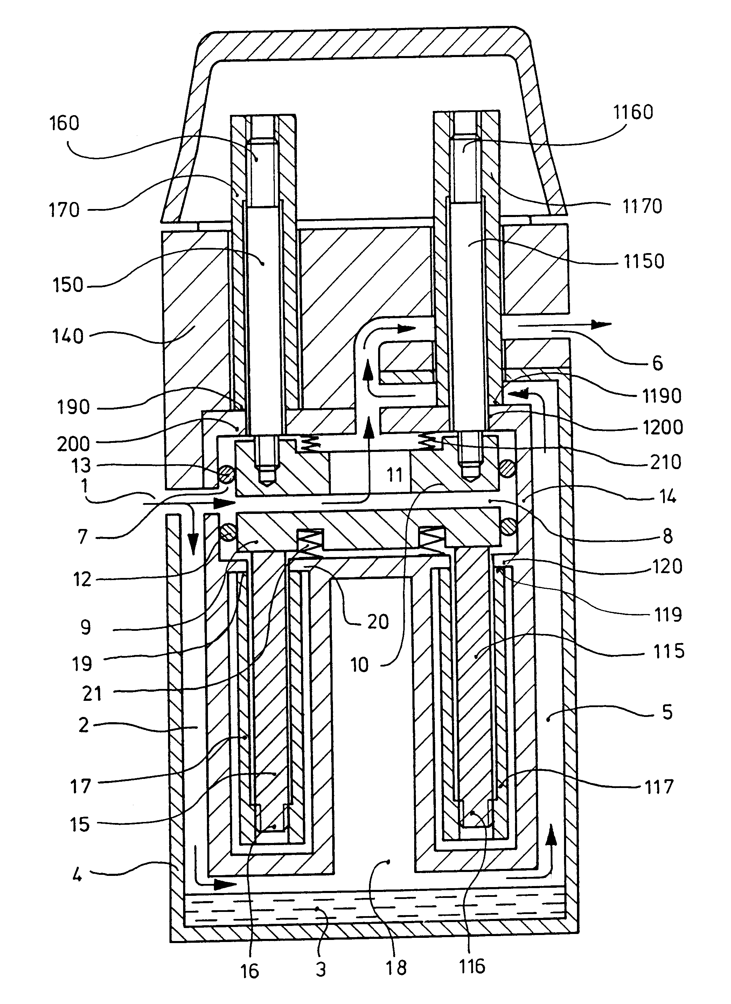

Ein Ausführungsbeispiel der Erfindung wird anhand der Zeichnung erläutert. Die einzige Figur zeigt die Vorrichtung zur Drosselung im Schnitt, eingesetzt in einen Narkosemittelverdunster.

Das zu dosierende Gas strömt durch den Einlaß (1) und teilt sich in zwei Teilströme. Der eine fließt durch den Kanal (2) abwärts bis zum Narkosemittel (3), das sich in dem Narkosemittelbehälter (4) befindet, überstreicht die Narkosemitteloberfläche und sättigt sich dabei mit dem Narkosemittel. Das gesättigte Gas steigt dann durch den Kanal (5) auf und verläßt den Narkosemittelverdunster durch den Auslaß (6). Der andere Teilstrom des durch den Einlaß (1) einströmenden Gases gelangt in den ringförmigen Raum (7). Der Spalt (8), der von den Stirnseiten der ringförmigen Formkörper (9) und Gegenformkörper (10) gebildet wird, beeinflußt die Gasmenge, die in den Raum (11) und weiter zum Auslaß (6) fließt, in dem sie sich mit dem mit Narkosemittel gesättigten Teilstrom vermischt. Die Formkörper (9) und (10) sind radial mit O-Ring-Dichtungen (12) und (13) zur Umhüllung (14) hin abgedichtet. Der Formkörper (9) ist als Joch mit Stangen (15, 115) aus Invar verbunden und wird mit federnden Elementen (21) gegenüber der Hülsenabstützung (20,120) gehalten. Die Stangen (15, 115) sind an ihrem einen Befestigungsende (16, 116) mit Hülsen (17, 117) aus Messing verschraubt, und stützen sich an ihrem dem Befestigungsende (16, 116) entgegengesetzten Ende (19, 119) an einer Hülsenabstützung (20, 120) der Umhüllung (14) ab.

Bei Erwärmung dehnen sich die Messinghülsen (17, 117) in ihrer Länge wesentlich mehr aus als die Invarstangen (15, 115) und die aus nichtrostendem Stahl gefertigte Umhüllung (14). Als Folge davon erweitert sich der Drosselspalt (8) und die durchfließende Gasmenge erhöht sich. Damit wird der durch Temperaturerhöhung verstärkten Verdunstung des Narkosemittels (3) in dem Verdunstungsraum (18) des Narkosemittelverdunsters entgegengewirkt. Die Mischung der Gasmengen, die durch den Verdunstungsraum und durch den Drosselspalt (8) fließen, hat einen annähernd temperaturunabhängigen Gehalt an Narkosemittel.

Um die thermisch bedingte Spaltbreitenvariation zu erhöhen ist der Gegenformkörper (10) mit einer zu der Verstelleinrichtung des Formkörpers (9) äquivalenten Verstelleinrichtung versehen.

Der Gegenformkörper (10) ist als Joch mit Stangen (150, 1150) aus Invar verbunden und wird mit federnden Elementen (210) gegenüber der Hülsenabstützung (200, 1200) gehalten. Die Stangen (150, 1150) sind an ihrem einen Befestigungsende (160, 1160) mit Hülsen (170, 1170) aus Messing verschraubt, und stützen sich an ihrem dem Befestigungsende (160, 1160) entgegengesetzten Ende (190, 1190) an einer Hülsenabstützung (200, 1200) des Einstellteils (140) ab.

## Patentansprüche

1. Narkosemittelverdunster mit einer Drosselung des ihn durchströmenden Gases, bei welcher ein Drosselspalt (8) in Abhängigkeit von der Umgebungstemperatur durch eine Kompensationseinrichtung verstellbar ist, welche einen Stangenkörper (15, 115) aus Material mit geringem Wärmeausdehnungskoeffizienten besitzt, der in einer Hülse (17, 117) aus Material mit großem Wärmeausdehnungskoeffizienten aufgenommen ist, wobei der Stangenkörper an seinem einen Ende (16, 116) mit der Hülse verbunden und an seinem anderen Ende (19, 119) mit einem Formkörper (9), der zusammen mit einem Gegenformkörper (10) den Drosselspalt bildet, versehen ist, dadurch gekennzeichnet, daß die Hülse (17, 117) von einer Umhüllung (14) aus korrosionsbeständigem Material umgeben ist, welche gegenüber den das Narkosegas führenden Durchströmungskanälen (2, 5, 6) gasdicht abgeschlossen ist, und daß die Hülse (17, 117) an ihrem dem Formkörper (9) zugewandten Ende (19, 119) auf einer Hülsenabstützung (20, 120) der Umhüllung (14) ruht.

2. Vorrichtung zur Drosselung eines Gasstromes nach Anspruch 1, dadurch gekennzeichnet, daß als Material mit geringem Wärmeausdehnungskoeffizienten eine Nickel-Eisen-Legierung vorgesehen ist.

3. Vorrichtung zur Drosselung eines Gasstromes nach Anspruch 1 oder 2, dadurch gekennzeichnet, daß als Material mit hohem Wärmeausdehnungskoeffizienten Messing vorgesehen ist.

4. Vorrichtung zur Drosselung eines Gasstromes nach einem der Ansprüche 1 bis 3, dadurch gekennzeichnet, daß der Gegenformkörper (10), äquivalent zum Formkörper (9) ebenfalls mit einer thermischen Verstelleinrichtung (140, 150, 160, 170, 190, 200, und 1150, 1160, 1170, 1190, 1200) versehen ist.

5. Vorrichtung nach einem der Ansprüche 1 bis 4, dadurch gekennzeichnet, daß mindestens zwei Stangen (15, 115) jeweils in einer Hülse (17, 117) aufgenommen und einerseits an ihrem jeweiligen Befestigungsende (16, 116) miteinander formschlüssig verbunden sind, und daß ihr anderes Ende an dem ein Joch für die beiden Stangen (15, 115) bildenden Formkörper (9) befestigt ist, und daß die Hülsen (17, 117) auf den zugehörigen Hülsenabstützungen (20, 120) der Umhüllung (14) ruhen.

## Claims

1. Vaporiser for anaesthetic solutions with restriction of the gas flowing therethrough, having a throttle gap (8) which can be adjusted in response to ambient temperature using a compensating device which has a bar-type body (15, 115) of material with a low coefficient of thermal expansion which is accommodated in a sleeve (17, 117) of material with a high coefficient of thermal expansion, the bar-type body being joined at one end (16, 116) to the sleeve and being provided at the other end (19, 119) with a moulded part (9) which together with a mating moulded part (10) forms the throttle gap, characterised in that the sleeve (17, 117) is enclosed by an envelope (14) of corrosion-resistant material which is sealed off in gas-tight manner with respect to the throughflow ducts (2, 5, 6) carrying the anaesthetic gas, and in that the sleeve (17, 117) rests with its end (19, 119) nearest the moulded part (9) on a sleeve support (20, 120) of the envelope (14).

2. Apparatus for restricting a flow of gas according to claim 1, characterised in that a nickel and iron alloy is the material with the low coefficient of thermal expansion.

3. Apparatus for restricting a flow of gas according to claim 1 or 2, characterised in that brass is the material with the high coefficient of thermal expansion.

4. Apparatus for restricting a flow of gas according to any of claims 1 to 3, characterised in that the mating moulded part (10) corresponding to the moulded part (9) is likewise provided with a thermal adjustment device (140, 150, 160, 170, 190, 200, and 1150, 1160, 1170, 1190, 1200).

5. Apparatus according to any of claims 1 to 4, characterised in that at least two bars (15, 115) are each accommodated in a sleeve (17, 117) and on the one hand are positively interconnected by their respective attachment end (16, 116), and in that their other end is fastened to the moulded part (9) which constitutes a yoke for the two bars (15, 115), and in that the sleeves (17, 117) rest on the associated sleeve supports (20, 120) of the envelope (14).

## Revendications

1. Evaporateur d'anesthésique pourvu d'un dispositif d'étranglement du gaz le traversant, dans lequel une fente d'étranglement (8) peut être réglée en fonction de la température ambiante grâce à un dispositif compensateur, possédant un corps en forme de tige (15, 115) constitué en un matériau présentant un faible coefficient de dilatation thermique, logé dans un manchon (17, 117) constitué en un matériau présentant un coefficient de dilatation thermique élevé, le corps en forme de tige étant lié, par l'une de ses extrémités (16, 116), au manchon, et étant lié, par l'autre extrémité (19, 119), à un corps profilé (9) délimitant une fente d'étranglement avec un corps profilé opposé (10); caractérisé en ce que le manchon (17, 117) est entouré d'une enveloppe (14) en matériau résistant à la corrosion, isolée de façon étanche aux gaz par rapport aux canaux d'écoulement (2, 5, 6) transportant le gaz anesthésique, et en ce que le manchon (17, 117), par son extrémité (19, 119) orientée vers le corps profilé (9), repose sur un support de manchon (20, 120) de l'enveloppe (14).

2. Dispositif d'étranglement d'un courant gazeux selon la revendication 1, caractérisé en ce qu'un alliage de nickel et de fer est prévu comme matériau présentant un faible coefficient de dilatation thermique.

3. Dispositif d'étranglement d'un courant gazeux selon la revendication 1 ou 2, caractérisé en ce que du laiton est prévu comme matériau présentant un coefficient de dilatation thermique élevé.

4. Dispositif d'étranglement d'un courant gazeux selon l'une des revendications 1 à 3, caractérisé en ce que le corps profilé opposé (10), comme le corps profilé (9), est pourvu d'un dispositif de réglage (140, 150, 160, 170, 190, 200 et 1150, 1160, 1170, 1190, 1200) commandé par la température.

5. Dispositif selon l'une des revendications 1 à 4, caractérisé en ce qu'au moins deux tiges (15, 115) sont, chacune, logées dans un manchon (17, 117) et sont, d'une part, assemblées par complémentarité de formes, par leurs extrémités de fixation (16, 116) respectives, en ce que leur autre extrémité est fixée au corps profilé (9) formant un pont pour les deux tiges (15, 115), et en ce que les manchons (17, 117) reposent sur les appuis de manchons (20, 120) correspondants de l'enveloppe (14).
